# EUROPEAN PATENT SPECIFICATION

(11) **EP 3 449 854 B1**
(45) Date of publication and mention of the grant of the patent: **08.07.2020**
(21) Application number: 18194949.6
(22) Date of filing: 14.03.2014
(51) Int. Cl.: A61B 17/66, A61B 17/84

(54) **ORTHOPEDIC COMPRESSION/DISTRACTION DEVICE**
ORTHOPÄDISCHE KOMPRESSIONS-/DISTRAKTIONSVORRICHTUNG
DISPOSITIF DE DISTRACTION/COMPRESSION ORTHOPÉDIQUE

(43) Date of publication of application: 06.03.2019
(62) Divisional of application: 14836955.6
(73) Proprietor: Wright Medical Technology, Inc., Memphis, TN 38117 (US)
(72) Inventor: THOREN, Brian, Memphis, Tennessee 38122 (US); MCCORMICK, Daniel, North Kingstown, RI 02852 (US); HARNESS, David, Eads, Tennessee 38028 (US); REED, Wesley, Memphis, TN 38117 (US); CRAMER, Thomas, Gainesvulle, Florida 32605 (US); LOWERY, Gary, Eads, Tennessee 38028 (US)
(74) Representative: Hirsch & Associés

(56) References cited:
- US-A- 5 549 608
- US-A1- 2006 247 649
- US-A1- 2014 012 269

## Description

### FIELD OF THE INVENTION

The present disclosure relates to an orthopedic device that can be used for compression or distraction of bone parts.

### BACKGROUND

Orthopedic devices utilizing elongated pins as fasteners for compression or distraction of bone parts finds many uses for treating orthopedic patients. "Elongated pins" will be used herein to refer to various pins and wires, such as K-wires, used for fixating bone parts or providing anchors. Therefore, there is a continuing need for an improved orthopedic device that expands the scope and ability of the orthopedic surgeons in treating patients in a variety of conditions.

Document US2006247649 A1 discloses an exemplary orthopedic device for compression or distraction of bone parts.

### SUMMARY

According to an aspect of the present disclosure an orthopedic device that can be used for compression or distraction of bone parts is described. The orthopedic device comprises an elongated body having first and second ends, a first arm member attached to and transversely extending away from said first end and terminating at an outer end and a second arm member transversely extending away from said elongated body and having a base portion and an outer end. The base portion is configured and adapted to movably engage the elongated body allowing the second arm member to be longitudinally movable along said elongated body. Said second arm member extends from the elongated body in the same direction as the first arm member. The orthopedic device also includes a locking sleeve hingeably connected to the outer end of each of the first and second arm members by a hinge joint, wherein said each locking sleeve is configured for lockably receiving an elongated pin. The locking sleeve comprises an elongated shaft having an elongated pin receiving bore extending therethrough and a collet provided at one end of the elongated shaft. The collet comprises a plurality of collet arms provided with screw threads integrally formed on their exterior surfaces. The locking sleeve also includes a collet nut that is threadably engaged to the collet for locking an elongated pin that is received in the elongated pin receiving bore.

The screw threads on the exterior surfaces of the collet arms are interrupted with a band of thread-free zone and the collet nut is provided with one or more detents protruding into the band of thread-free zone and providing a mechanical interference that prevents the collet nut from being removed from the threaded engagement with the collet.

According to another aspect, a locking sleeve for lockably receiving an elongated pin is also disclosed. The locking sleeve comprises an elongated shaft having an elongated pin receiving bore extending therethrough, a collet provided at one end of the elongated shaft, where the collet comprises a plurality of collet arms provided with screw threads integrally formed on their exterior surfaces, and a collet nut that is threadably engaged to the collet for locking an elongated pin that is received in the elongated pin receiving bore.

### BRIEF DESCRIPTION OF THE DRAWINGS

**FIG. 1** is an orthographic view of an orthopedic device according to an aspect of the present disclosure.
**FIG. 2** is another orthographic view of the orthopedic device of **FIG. 1****.**
**FIG. 3** is an exploded orthographic view of locking sleeves according to an aspect of the present disclosure.
**FIG. 4** is an elevation view of the locking sleeves with the collet nut on one of the locking sleeves cross-sectioned.
**FIG. 5** is an exploded orthographic view showing the threaded collet end of a locking sleeve and a collet nut where the collet nut is shown in cross-section.
**FIG. 6** shows an example of a pin Spanner wrench that can be used to turn the collet nut according to one embodiment.
**FIG. 7** shows a detailed view of a collet nut according to another embodiment.

The features shown in the above referenced drawings are illustrated schematically and are not intended to be drawn to scale nor are they intended to be shown in precise positional relationship. Like reference numbers indicate like elements.

### DETAILED DESCRIPTION

This description of the exemplary embodiments is intended to be read in connection with the accompanying drawings, which are to be considered part of the entire written description. In the description, relative terms such as "lower," "upper," "horizontal," "vertical,", "above," "below," "up," "down," "top" and "bottom" as well as derivative thereof (e.g., "horizontally," "downwardly," "upwardly," etc.) should be construed to refer to the orientation as then described or as shown in the drawing under discussion. These relative terms are for convenience of description and do not require that the apparatus be constructed or operated in a particular orientation. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise.

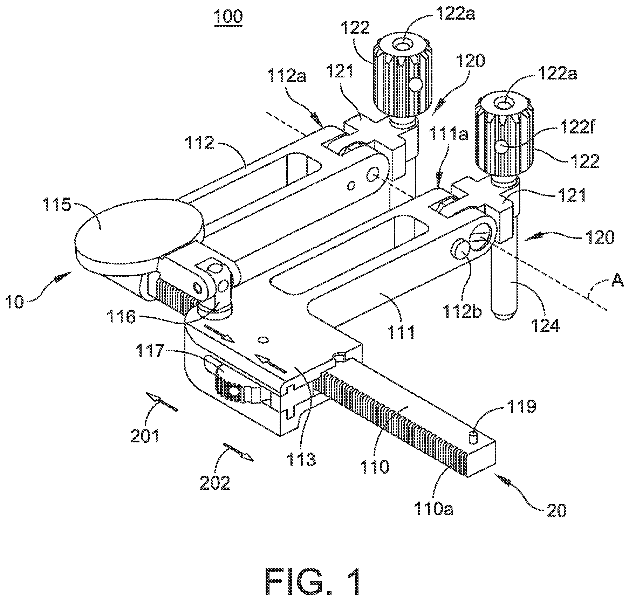

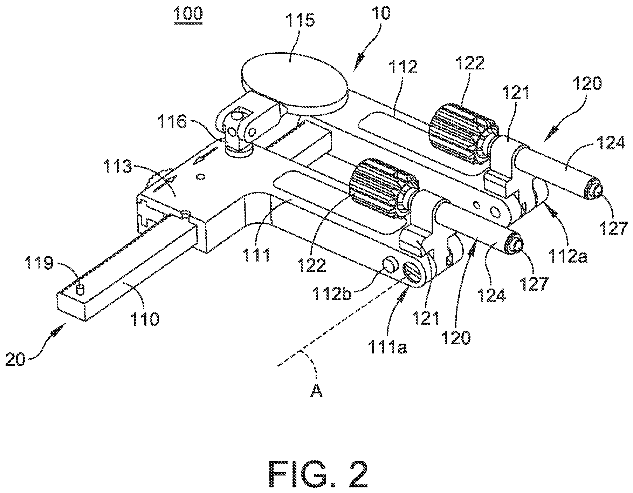

FIGS. 1-2 show an orthopedic device **100** that can be used for compression or distraction of bone parts according to an aspect of the present disclosure. The orthopedic device **100** comprises an elongated body **110** having first end **10** and a second end **20.** A first arm member **112** extends away from the first end **10** in a direction transverse to the elongated body **110** and terminates at an outer end **112a.** The first arm member **112** can be integrally formed with the body **110** or otherwise attached to the body **110.** A second arm member **111** transversely extends away from the elongated body **110** and terminates at an outer end **111a.** The second arm member **111** is configured with a base portion **113** that is configured and adapted to movably engage the elongated body **110** allowing the second arm member **111** to be longitudinally movable along the elongated body.

According to one embodiment, the elongated body **110** is provided with ratchet teeth **110a** along its length for engaging with the base portion **113.** The base portion **113** is configured with ratcheting mechanisms that can selectably operate in compression or distraction mode. For example, the base portion **113** can be configured with a mode selector switch **117** that can be used to switch between the compression mode and the distraction mode.

The ratcheting mechanism is configured so that the selector switch 117 not only changes the direction of the base portion's movement but also changes the locking direction of the device. This allows the desired compression or distraction to be maintained once it is reached. In the compression mode, the second arm member **111** is locked from moving in the distraction direction **202** and can only move in the compression direction **201.** Conversely, in the distraction mode, the second arm member **111** is locked from moving in the compression direction **201** and can only move in the distraction direction **202.**

The base portion **113** also can be configured to have a neutral position for the selector switch **117** which will allow the second arm member **111** to freely move along the elongated body **110.** The second arm member **111** is moved by turning a turning key handle **115** which is connected to a shaft **116** that actuates the internal mechanism of the base portion **113.** The internal structure of the base portion **113,** although not shown, is configured with a ratcheting mechanism utilizing a rack and pinion type gear arrangement or other suitable mechanisms known to one skilled in the art.

The elongated body **110** is provided with an appropriate mechanism for stopping the base portion **113** from sliding off the second end **20** of the elongated body **110.** For example, in the embodiment shown in FIG. 1, a stop pin **119** provided near the second end **20** of the elongated body serves that function.

The second arm member **111** extends from the elongated body **110** in the same direction as the first arm member **112.** The orthopedic device **100** also includes a locking sleeve **120** hingeably connected to the outer end **112a, 111a** of each of the first and second arm members **112, 111** by a hinge joint **121,** wherein each locking sleeve **120** is configured for lockably receiving an elongated pin (not shown).

The locking sleeve **120** comprises an elongated shaft **124** having an elongated pin receiving bore **127** extending therethrough. Each of the hinge joints **121** hingeably connects one locking sleeve **120** to the outer end of one of the arm members **112, 111.** The hinge axes of the hinge joints **121** are coaxially aligned and represented in FIG 1 by a hinge axis A.

According to an embodiment, the hinge joints **121** can be configured and adapted to be normally locked in a position and prevented from pivoting about the hinge axis **A** by a spring-loaded locking pin **112b.** When the spring-loaded locking pin **112b** is pressed, the hinge joint **121** is unlocked and free to pivot about the hinge axis **A.**

Each of the locking sleeves **120** is connected to the hinge joint **121** so that the elongated shaft **124** and thus the elongated pin receiving bore **127** is oriented transverse to the hinge axis **A.** The transverse orientation refers to the fact that the elongated shaft **124** and the elongated pin receiving bore **127** are at right angle to the hinge axis **A.**

According to an embodiment, the locking sleeve **120** further comprises a threaded collet **220** provided at one end of the elongated shaft **124** and a collet nut **122** that is threadably engaged to the threaded collet **220** for locking an elongated pin that is received in the elongated pin receiving bore **127.** The threaded collet **220** comprises a plurality of collet arms **221** defining one end of the elongated pin receiving bore **127.** The collet arms **221** are provided with screw threads **227** integrally formed on their exterior surfaces whereby said collet nut **122** threadably engages the collets for locking the elongated pin received in the elongated pin receiving bore **127.** The integration of the screw threads **227** into the collet arms **221** allow for a low profile, compact design of the collet **220.** The collet arms **221** are defined by a plurality of slots **222.**

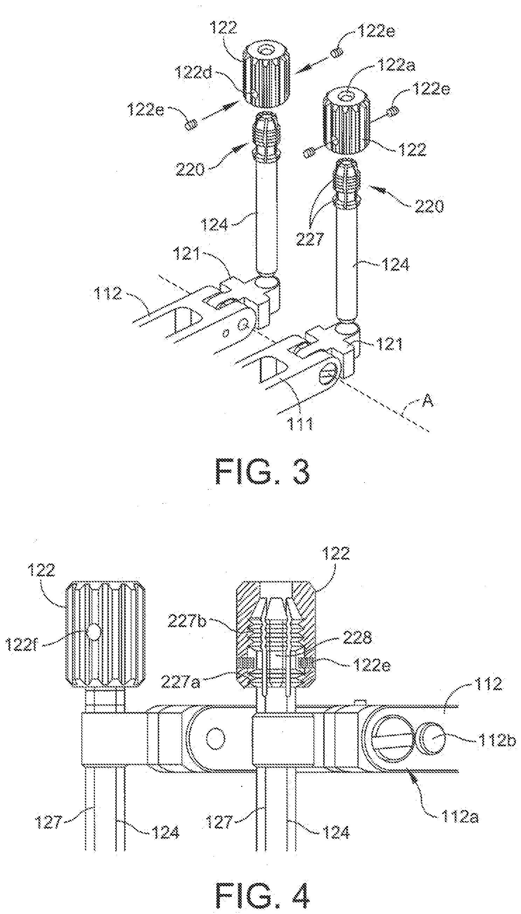

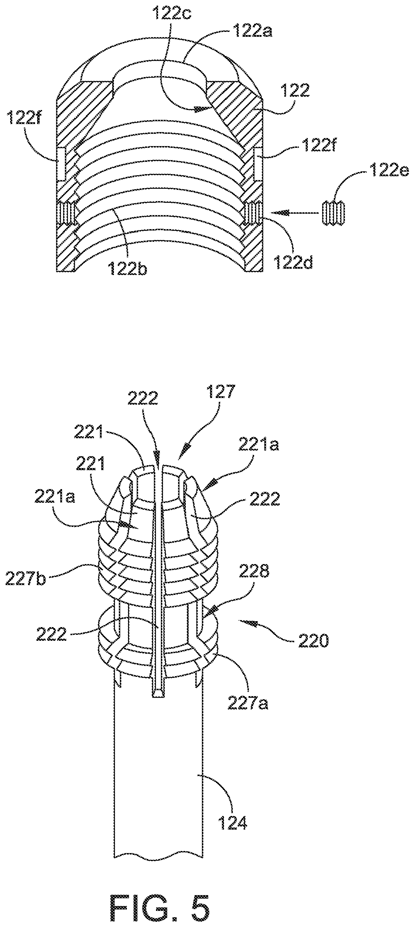

FIGS. 3-5 show detailed structures of the threaded collet **220** and the collet nut **122.** As shown in the longitudinal cross-section view of the collet nut **122** in FIG 5, the collet nut **122** is open at one end for receiving the threaded collet **220** and has an interior surface provided with screw threads **122b** for threadably engaging the screw threads **227** of the collet **220.** At the end opposite from the threaded collet receiving end, a through hole **122a** is provided for the elongated pin received in the elongated pin receiving bore **127.** The interior surface of the collet nut **122** is configured with a conical surface **122c.** When the collet nut **122** is fully threaded onto the threaded collet **220,** the conical surface **122c** contacts the collet arms **221** and presses against the chamfered surfaces **221a** of the collet arms **221** and pushes the collet arms **221** radially inwards. When an elongated pin is received in the elongated pin receiving bore **127,** the threading action of the collet nut **122** causes the collet arms **221** to move radially inward and clamp onto the elongated pin and lock the elongated pin in place.

According to an aspect of the present disclosure, the screw threads **227** on the exterior surfaces of the collet arms **221** are interrupted with a band of thread-free zone **228** dividing the screw threads **227** into a lower portion **227a** and an upper portion **227b** as shown in FIGS. 4 and 5. The collet nut **122** is provided with one or more detents **122e** protruding into the band of thread-free zone **228** and thus providing a mechanical interference with the upper portion **227b** of the screw threads that prevents the collet nut **122** from being removed from the threaded engagement with the collet **220.** If one tries to remove the collet nut **122,** the one or more detents **122e** and the upper portion **227b** of the screw threads would interfere with each other and prevent the collet nut 122 from being removed.

In one embodiment, the one or more detents **122e** are set screws and one or more set screw receiving holes **122d** are provided on the side wall of the collet nut **122.** The set screw receiving holes **122d** are threaded so that a set screw detents **122e** can be threaded therein. During assembly of the locking sleeve **120,** the collet nut **122** is threaded onto the collet **220** until the set screw receiving holes **122d** align with the thread-free zone **228.** Then the set screw detents **122e** are threaded into the receiving holes **122d** until the detents **122e** protrude into the thread-free zone **228.** In FIG. 4, the sectioned view of one of the collet nut **122** shows this arrangement. The detents **122e** can be provided in many other forms than set screws. For example, the detents **122e** can be dowel pins of appropriate length that are either press fit, welded or secured in any suitable manner into receiving holes **122d** in the collet nut **122.**

Generally, it is envisioned that the user can lock an elongated pin received in the elongated receiving bore **127** by tightening the collet nut **122** by hand. According to another aspect of the present disclosure, however, the collet nut **122** can be configured and adapted to accommodate a driver tool for assisting the user in turning and tightening the collet nut.

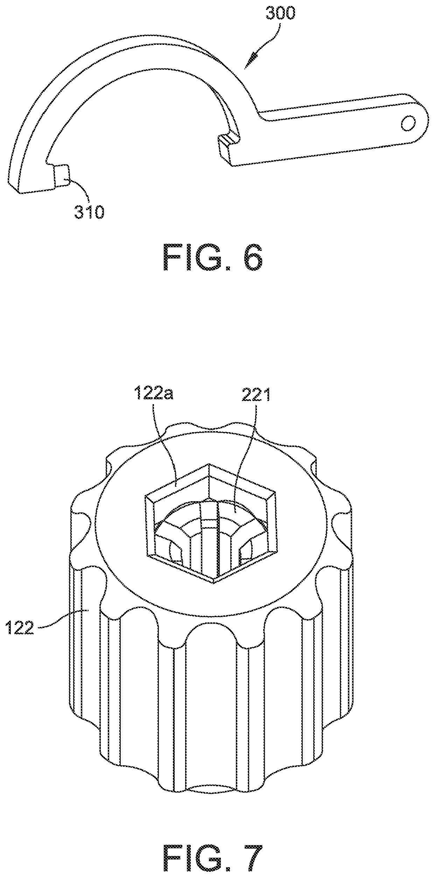

In one example, the driver tool can be a pin spanner wrench **300** shown in FIG. 6 and the collet nut is provided with one or more holes or recesses **122f** (see FIGS. 1, 4 and 5) along the outer surface of the collet nut for engaging the pin **310** of the driver tool. FIG. 7 shows another embodiment of the collet nut **122** that is configured to be tightened using a cannulated driver tool (not shown) for locking/tightening the collet nut **122.** The hollow tubular opening of the cannulated driver tool would accommodate the elongated pin that is positioned in the elongated pin receiving bore **127** of the locking sleeve **120.** The through hole **122a** of the collet nut can be configured in any variety of shapes as long as it is large enough to allow the elongated pin to pass through it. In this embodiment, the through, hole **122a** would be provided in a shape that matches the particular driving feature of the driver. For example, if the driver is a hex, square, or star driver, the through hole **122a** would be appropriately shaped to engage the driving feature of the driver. In the example shown in FIG. 7, the through hole **122a** has a hexagon shape for accommodating a hex tip driver.

As described, the orthopedic device **100** of the present disclosure is a universal device that can be used for compression or distraction of bone parts that are secured to the first and second arm members **112, 111** by elongated pins, such as K-wires, locked into the elongated pin receiving bores **127** of the locking sleeves **120.** Referring to FIG. 1, after the bone parts are secured, the movable second arm member **111** can be moved in compression direction **201** or distraction direction **202** by turning the turning key handle **115.**

Although the invention has been described in terms of exemplary embodiments, it is not limited thereto. Rather, the appended claims should be construed broadly, to include other variants and embodiments of the invention, which may be made by those skilled in the art without departing from the scope of the invention. The scope of the invention disclosed herein is to be limited only by the following claims.

## Claims

1. An orthopedic device (100) comprising:
an elongated body (110) having first and second ends;
a first arm member (112) attached to and extending away from said first end and terminating at an outer end;
a second arm member (111) extending from said elongated body in the same direction as the first arm member and having a base portion and an outer end, wherein the base portion is configured and adapted to engage the elongated body (110) and allow the second arm member (111) to be longitudinally movable along said elongated body (110); and
a locking sleeve (120) hingeably connected to the outer end of each of the first and second arm members by a hinge joint, wherein said locking sleeve (120) comprises:
an elongated shaft (124) having an elongated pin receiving bore (127) extending therethrough;
a collet (220) provided at one end of the elongated shaft (124), said collet (220) comprising a plurality of collet arms (221) provided with screw threads integrally formed on their exterior surfaces; and
a collet nut (122) having an interior surface provided with screw threads for threadably engaging the screw threads (227) of the collet (220);
wherein rotation of the collet nut (122) in a first direction causes the plurality of collet arms to move radially inward for locking an elongated pin that is received in the elongated pin receiving bore.

2. The orthopedic device of claim 1, wherein said screw threads on the exterior surfaces of the collet arms (221) are interrupted with a band of thread-free zone and the collet nut (122) is provided with one or more detents protruding into the band of thread-free zone and providing a mechanical interference that prevents the collet nut (122) from being removed from the threaded engagement with the collet (220).

3. The orthopedic device of claim 2, wherein said one or more detents are set screws that are threaded into the collet nut (122).

4. The orthopedic device of claim 3, further comprising a spring loaded locking pin (112b) that is configured to lock rotation of the hinge joint.

5. The orthopedic device of claim 4, wherein each of said hinge joints having a hinge axis and said hinge axes are in axial alignment.

6. The orthopedic device of either claim 4 or 5, further wherein each of the locking sleeves (120) is connected to the hinge joint so that the elongated pin receiving bore (127) is oriented transverse to the hinge axes.

7. The orthopedic device of any preceding claim, wherein said collet nut (122) is configured and adapted for accommodating a driver tool for turning the collet nut (122).

8. The orthopedic device of claim 7. wherein said collet nut (122) is provided with one or more recesses on its outer surface for accommodating a pin spanner wrench type driver tool.

9. The orthopedic device of claim 8, wherein said collet nut (122) is provided with a through hole for accommodating the elongated pin that is received in the elongated pin receiving bore (127) and said through hole has an outline that is shaped to match the driver tool's driving feature.

10. The orthopedic device of any preceding claim, wherein the collet nut (122) includes an inner surface (122c) that is at least partially conical and is configured to engage a chamfered surface (221a) of each of the plurality of collet arms (221) to facilitate radially inward movement of the collet arms (221).

## Patentansprüche

1. Orthopädische Vorrichtung (100), umfassend:
einen länglichen Körper (110) mit einem ersten und zweiten Ende;
ein erstes Armelement (112), das an dem ersten Ende angebracht ist und sich von diesem weg erstreckt und an einem äußeren Ende endet;
ein zweites Armelement (111), das sich von dem länglichen Körper in derselben Richtung wie das erste Armelement erstreckt und einen Basisabschnitt und ein äußeres Ende aufweist, wobei der Basisabschnitt ausgelegt und geeignet ist, mit dem länglichen Körper (110) in Eingriff zu gelangen und es dem zweiten Armelement (111) zu gestatten, in Längsrichtung entlang des länglichen Körpers (110) bewegbar zu sein; und
eine Verriegelungsmanschette (120), die mit dem äußeren Ende jedes von dem ersten und zweiten Armelement durch eine Scharnierverbindung scharnierverbunden ist, wobei die Verriegelungsmanschette (120) umfasst:
einen länglichen Schaft (124) mit einer länglichen Stiftaufnahmebohrung (127), die sich durch diesen erstreckt;
eine Hülse (220), die an einem Ende des länglichen Schafts (124) bereitgestellt ist, wobei die Hülse (220) eine Vielzahl von Hülsenarmen (221), welche mit Schraubengewinden versehen sind, die einteilig an ihren Außenflächen gebildet sind, umfasst; und
eine Hülsennut (122) mit einer Innenfläche, die mit Schraubengewinden versehen ist, um mit den Schraubengewinden (227) der Hülse (220) in Gewindeeingriff zu gelangen;
wobei eine Drehung der Hülsennut (122) in einer ersten Richtung bewirkt, dass sich die Vielzahl von Hülsenarmen radial einwärts bewegt, um einen länglichen Stift, der in der länglichen Stiftaufnahmebohrung aufgenommen wird, zu verriegeln.

2. Orthopädische Vorrichtung nach Anspruch 1, wobei die Schraubengewinde an den Außenflächen der Hülsenarme (221) mit einem Band einer gewindefreien Zone unterbrochen sind, und die Hülsennut (122) mit einem oder mehreren Vorsprüngen versehen ist, die in das Band der gewindefreien Zone vorstehen und eine mechanische Interferenz liefern, die verhindert, dass die Hülsennut (122) aus dem Gewindeeingriff mit der Hülse (220) entfernt wird.

3. Orthopädische Vorrichtung nach Anspruch 2, wobei der eine oder die mehreren Vorsprünge Stellschrauben sind, die in die Gewindenut (122) geschraubt sind.

4. Orthopädische Vorrichtung nach Anspruch 3, umfassend einen federbeaufschlagten Verriegelungsstift (112b), der ausgelegt ist, die Drehung des Scharniergelenks zu verriegeln.

5. Orthopädische Vorrichtung nach Anspruch 4, wobei jede der Scharnierverbindungen eine Scharnierachse aufweist, und die Scharnierachsen axial ausgerichtet sind.

6. Orthopädische Vorrichtung nach einem der Ansprüche 4 oder 5, wobei ferner jede der Verriegelungsmanschetten (120) mit der Scharnierverbindung derart verbunden ist, dass die längliche Stiftaufnahmebohrung (127) quer zu den Scharnierachsen ausgerichtet ist.

7. Orthopädische Vorrichtung nach einem der vorhergehenden Ansprüche, wobei die Hülsennut (122) ausgelegt und geeignet ist, ein Schraubwerkzeug zum Drehen der Hülsennut (122) aufzunehmen.

8. Orthopädische Vorrichtung nach Anspruch 7, wobei die Hülsennut (122) mit einer oder mehreren Vertiefungen an ihrer Außenfläche versehen ist, um ein Schraubwerkzeug vom Typ eines Stiftspannschlüssels aufzunehmen.

9. Orthopädische Vorrichtung nach Anspruch 8, wobei die Hülsennut (122) mit einem Durchgangsloch versehen ist, um den länglichen Stift aufzunehmen, der in der länglichen Stiftaufnahmebohrung (127) aufgenommen ist, und das Durchgangsloch einen Umriss hat, der geformt ist, um mit dem Schraubmerkmal des Schraubwerkzeugs zusammenzupassen.

10. Orthopädische Vorrichtung nach einem der vorhergehenden Ansprüche, wobei die Hülsennut (122) eine Innenfläche (122c) aufweist, die mindestens teilweise konisch ist und ausgelegt ist, mit einer abgeschrägten Fläche (221a) der Vielzahl von Hülsenarmen (221) in Eingriff zu gelangen, um eine Bewegung der Hülsenarme (221) radial nach innen zu erleichtern.

## Revendications

1. Dispositif orthopédique (100) comprenant :
un corps allongé (110) ayant des première et seconde extrémités ;
un premier élément de bras (112) fixé sur et s'étendant à distance de ladite première extrémité et se terminant par une extrémité externe ;
un second élément de bras (111) s'étendant à partir dudit corps allongé dans la même direction que le premier élément de bras et ayant une partie de base et une extrémité externe, dans lequel la partie de base est configurée et adaptée pour mettre en prise le corps allongé (110) et permettre au second élément de bras (111) d'être longitudinalement mobile le long dudit corps allongé (110) ; et
un manchon de verrouillage (120) raccordé, par charnière, à l'extrémité externe de chacun des premier et second éléments de bras par un joint de charnière, dans lequel ledit manchon de verrouillage (120) comprend :
un arbre allongé (124) ayant un alésage de réception de broche allongée (127) s'étendant à travers ce dernier ;
une pince (220) prévue au niveau d'une extrémité de l'arbre allongé (124), ladite pince (220) comprenant une pluralité de bras de pince (221) prévus avec des filetages de vis formés de manière solidaire sur leurs surfaces extérieures ; et
un écrou de pince (122) ayant une surface intérieure prévue avec des filetages de vis pour mettre en prise, par filetage, les filetages de vis (227) de la pince (220) ;
dans lequel la rotation de l'écrou de pince (122) dans une première direction amène la pluralité de bras de pince à se déplacer radialement vers l'intérieur pour verrouiller une broche allongée qui est reçue dans l'alésage de réception de broche allongée.

2. Dispositif orthopédique selon la revendication 1, dans lequel lesdits filetages de vis sur les surfaces extérieures des bras de pince (221) sont interrompus avec une bande de zone dépourvue de filetage et l'écrou de pince (122) est prévu avec une ou plusieurs détentes faisant saillie dans la bande de zone dépourvue de filetage et fournissant une interférence mécanique qui empêche l'écrou de pince (122) d'être retiré de la mise en prise filetée avec la pince (220).

3. Dispositif orthopédique selon la revendication 2, dans lequel lesdites une ou plusieurs détentes sont des vis de serrage qui sont filetées dans l'écrou de pince (122).

4. Dispositif orthopédique selon la revendication 3, comprenant en outre une broche de verrouillage à ressort (112b) qui est configurée pour verrouiller la rotation du joint de charnière.

5. Dispositif orthopédique selon la revendication 4, dans lequel chacun desdits joints de charnière ayant un axe de charnière et lesdits axes de charnière sont en alignement axial.

6. Dispositif orthopédique selon la revendication 4 ou 5, dans lequel en outre chacun des manchons de verrouillage (120) est raccordé au joint de charnière de sorte que l'alésage de réception de broche allongée (127) est orienté transversalement par rapport aux axes de charnière.

7. Dispositif orthopédique selon l'une quelconque des revendications précédentes, dans lequel ledit écrou de pince (122) est configuré et adapté pour loger un outil d'entraînement pour faire tourner l'écrou de pince (122).

8. Dispositif orthopédique selon la revendication 7, dans lequel ledit écrou de pince (122) est prévu avec un ou plusieurs évidements sur sa surface externe pour loger un outil d'entraînement de type clé à ergots.

9. Dispositif orthopédique selon la revendication 8, dans lequel ledit écrou de pince (122) est prévu avec un trou débouchant pour loger la broche allongée qui est reçue dans l'alésage de réception de broche allongée (127) et ledit trou débouchant a un contour qui est formé pour correspondre à la caractéristique d'entraînement de l'outil d'entraînement.

10. Dispositif orthopédique selon l'une quelconque des revendications précédentes, dans lequel l'écrou de pince (122) comprend une surface interne (122c) qui est au moins partiellement conique et est configurée pour mettre en prise une surface chanfreinée (221a) de chacun de la pluralité de bras de pince (221) pour faciliter le mouvement radialement vers l'intérieur des bras de pince (221).
